Europäisches Patentamt

European Patent Office

Office européen des brevets

(11) Publication number: 0 050 215
B1

(12) EUROPEAN PATENT SPECIFICATION

(45) Date of publication of patent specification: 19.11.87

(21) Application number: 81107408.7

(22) Date of filing: 18.09.81

(51) Int. Cl.⁴: **C 08 L 67/04,** A 61 L 17/00, C 08 G 63/06, C 08 G 63/08, C 08 G 63/42, C 08 G 63/76, C 08 K 3/22, C 08 K 3/24

(54) Modification of polyglycolic acid to achieve variable in-vivo physical properties.

(30) Priority: 20.10.80 US 198565
20.10.80 US 198566

(43) Date of publication of application:
28.04.82 Bulletin 82/17

(45) Publication of the grant of the patent:
19.11.87 Bulletin 87/47

(84) Designated Contracting States:
BE CH DE FR GB IT LI NL SE

(56) References cited:
EP-A-0 011 528
BE-A- 872 208
DE-A-2 742 128
FR-A-2 391 734
US-A-2 362 511
US-A-3 463 158
US-A-3 636 956
US-A-4 057 537
US-A-4 135 622
US-A-4 279 249

(73) Proprietor: AMERICAN CYANAMID COMPANY
1937 West Main Street P.O. Box 60
Stamford Connecticut 06904-0060 (US)

(72) Inventor: Ritter, Thomas Alexander
82 Treble Road
Bristol Connecticut (US)
Inventor: Kaganov, Alan Lawrence
150 Gary Road
Stamford Connecticut (US)
Inventor: Budris, John Peter
691 Farm Road
Cheshire Connecticut (US)

(74) Representative: Wächtershäuser, Günter, Dr.
Tal 29
D-8000 München 2 (DE)

**Description**

The invention relates to a method for preparing gastro-intestinal anastomosis devices or other surgical tubular support, implant or stenotic devices. Such devices are known from US—A—3636956 and US—A—4135622.

Unmodified polyglycolic acid maintains its integrity for about twenty eight days. In certain surgical uses, e.g. in the colon, the rate of healing is rapid. It is desirable for the surgical implant to disintegrate following the wound healing period.

An implanted surgical element may need a relatively high initial tensile strength followed by a relatively rapid loss of strength and/or disintegration rate. For example, in a gastro-intestinal anastomosis device, a relatively high initial tensile strength is required but disintegration and passage of device fragments should occur in about 8 to 15 days.

The method of the invention as claimed in claim 1 produces a surgical device, which satisfies this need.

The advantages of the modified polyglycolic acid devices produced by this invention are not known in the prior art. One advantage is the relatively closer control of the modified polyglycolic acid properties, specifically the strength and in-vivo degradation properties. The properties can be controlled by the use of various concentrations of barium sulfate as filler. The properties are further controlled by lowering the intrinsic viscosity of the filled polyglycolic acid. This is accomplished by subjecting the filled polyglycolic acid to hydrolytic degradation by boiling in distilled water. A range of tensile strengths and disintegration rates can thus be obtained. Thus, depending on the surgical need, e.g. a staple, a hemostat, a bowel anastomosis ring and the like, a surgeon has available a degradable polymer with a variable range of in-vivo physical properties. Besides tensile strength and degradation rate, the filler can also change the fracture properties, compression strength, elongation, elastic modulus and/or creep properties of polyglycolic acid for other surgical uses.

Another advantage of the modified polyglycolic acid (PGA) of this invention is the degradation pattern. The fragments of the filled PGA are relatively small, for example some fragments are on the order of magnitude of about one-sixteenth of an inch (1,6 mm). Also, the fractures particles are relatively soft and less sharp, thus reducing the risk of trauma to adjacent tissue. Barium sulfate does not significantly adhere to PGA and therefore tends to effect a uniform breakup of the modified PGA. The lack of adherence develops numerous points of weakness in the material. Also, physiological forces may tend to enhance the fracture pattern. Finally, by the use of the method of this invention, the physical properties of the filled PGA can be closely matched to the physiological requirements of the surgical procedure or repair.

In summary, the advantages of the modified PGA of this invention are a relatively closer control of the physical properties; a smaller particle on fracturing; and a softer particle on fracturing. These advantages are seen in specific surgical uses, e.g. in a gastro-intestinal anastomosis device and in a surgical staple, where the modified PGA can be used to manufacture an improved surgical element.

Still other advantages are obtained from the modified PGA. Another advantage is that the in-vivo strength of the modified PGA can be maintained up to at least 14 days. Still another advantage is that the physical properties of the modified PGA can be changed for tissues which heal rapidly, for example, in the bowel. A device can now be manufactured using the modified PGA which has relatively high tensile strength on implant but which rapidly loses its strength with time. The device then breaks down into beads and is either absorbed or passed by the body.

In a preferred embodiment the amount of filler is between 20 and 25% by weight.

One embodiment contains the additional step of subjecting the polymer or the mixture to irradiation degradation. Another embodiment contains the additional step of subjecting the polymer or the mixture to repelletizing.

The boiling time of about 30 minutes at about 100°C achieves the desired modified properties for many surgical uses.

If increased tensile strength is required, a coupling compound can be used to more firmly adhere the filler to the polyglycolic acid. Such a coupling compound is a silane. A coupling compound may be useful in hard tissue repair, e.g. in bone fractures, preferably with an absorbable filler.

The medical uses of the modified polyglycolic acid of this invention include, but are not necessarily limited to:

1: Solid products, molded or machined, such as:
a. Orthopedic prosthetics, e.g. pins, clamps, screws, intermedullary rods and compression plates
b. Clips
c. Staples
d. Hooks, buttons, snaps and fasteners
e. Bone substitutes (e.g. mandible prosthesis)
f. Tubular implants, stenotic devices or supports
2. Fibrillar Products, knitted or woven, including velours, such as:
a. Facial substitutes
b. Gauze, fabric, sheet, felt or sponge
c. Sutures, including ligatures

2

3. Miscellaneous, such as:
a. Foam as absorbable prosthesis
b. Substitute for wire in fixations
In combination with other components, such as:
1. Solid Products, molded or machines, e.g. reinforced bone pins, etc.
2. Fibrillar Products
The following examples more fully describe the mixture and method of this invention.

Example 1

The following example relates to the preparation of polyglycolic acid (PGA), or polyglycolic acid filled with 20%, 22.5%, 25% and 40% barium sulfate. PGA in pellet form can be injection molded without preprocessing steps 1. to 3.

1. Material preparations

1.1 Grinding
PGA in pellet form is ground into 2 mm particles. The ground PGA is then stored in plastic bags in a dry cabinet at 21°C with less than 50 ppm $H_2O$ until it is mixed with the $BaSO_4$.

1.2 Mixing
The ground PGA is mixed with $BaSO_4$ using conventional techniques for mixing powders.

1.3 Polymer drying
After mixing, the polymer blend is vacuum dried by maintaining a temperature of 120°C with a vacuum of less than 0,013 bar for six hours. Dry nitrogen with 283 l/h (standard conditions) is run through the polymer.

2. Melt blending
Additional mixing of the PGA/$BaSO_4$ blend is accomplished by melt blending the polymer using conventional techniques at a temperature of 270°C. The resultant blended material is then cooled to ambient temperature under dry conditions of less than 50 ppm $H_2O$ for 4 hours.

3. Granulation
After the blended material is cooled to ambient temperature, it is granulated into less than 5 mm particles, redried and vacuum sealed in cups.

4. Molding conditions
The PGA or PGA/$BaSO_4$ blend is injection molded into bowel anastomosis rings using conventional molding techniques.

Typical molding conditions are:

| | |
|---|---|
| Temperature: | 235°C |
| Pressure: | 40—70 bar (600—1000 psi) |
| Cycle time: | 1 min. |
| Injection time: | 55 sec |
| Molding time: | 10—15 sec. |

5. Post molding treatments
The molded device is subjected to the following post treatment conditions.

5.1 Annealing
110°C with a vacuum of less than 1 mm. of Hg $1,3 \cdot 10^{-3}$ bar for 3 hours.

5.2 Etching
Placed in boiling $H_2O$ for 30 minutes, cooled and then dried in methanol for 2 1/2 hours, followed by drying in a vacuum oven at 50°C and less than 1 mm. of Hg $1,3 \cdot 10^{-3}$ bar for 30 minutes.

6. Sterilization
The packaged post treated device is subjected to a gas chamber sterilization employing a sterilant mixture containing ethylene oxide with a diluent such as freon. A typical sterilization cycle is described below:

| Temperature | 30°C |
| Pre-vacuum | 0,87 bar |
| RH | 20% |
| Gas | EO/Freon in a 12/88 ratio |
| Pressure | 2,4 bar (20 PSIG) |
| EO Concentration | 11100 mg/L |
| Exposure | 7 hours |
| Post vacuum | 0,87 bar (26″ Hg) |

Example 2

Various samples of a bowel anastomosis ring (BAR) were prepared using the mixtures of Example 1. The hydrolytic treatment was a 30 minute distilled water boil. All of the samples were 28 mm in diameter. The percent barium sulfate and implant subject were as follows:

| Sample No. | % BaSO$_4$ | Implant subject |
| --- | --- | --- |
| 1 | 22.5 | Beagle |
| 2 | 22.5 | " |
| 3 | 22.5 | " |
| 4 | 22.5 | " |
| 5 | 22.5 | " |
| 6 | 40 | " |
| 7 | 40 | Foxhound |
| 8 | 25 | " |

Procedure

Prior to anesthetic inducation, the animals received an enema and routine preinduction sedication. Under aseptic conditions a low midline laparotomy was made. The descending colon was mobilized and the mesenteric blood vessels supplying a selected segment were doubly ligated and transected. Purse-string sutures were placed proximally and distally to the devascularized colon segment using two purse-string bowel clamps that had been appropriately placed. The colon segment between the clamps was resected flush with the clamps and the segment was discarded. For implantation of the BAR, the proximal clamp was removed first and the colonic stoma triangulated, cleansed with warm saline and dilated. One ring of the BAR was inserted into the proximal stoma and the purse-string suture tied to secure the colon to the BAR struts. This procedure was then repeated to insert the other BAR ring into the distal stoma. Care was taken to align the mesentery during the BAR insertion procedures. The BAR rings were closed (forced closer together) using finger pressure on the colon serosa. The anastomosis was inspected to insure adequate serosa to serosa to serosa approximation around the circumference of the BAR. When necessary, stiches were taken to insure the adequacy of the purse-string suture and/or the approximation of the serosa to serosa union.

When the anastomoses were completed, the colons were replaced and the laparotomies closed in three layers. The dogs were returned to cages and treated with routine post-operative antibiotics. The Beagles were given milk only on the first and second post-operative days. From days 3 to 17, they were fed canned dog food mixed with milk. Water was available at all times. Fecal material were fluoroscoped daily, except weekends. All five Beagles were sacrificed on the 17th post-operative day and anastomotic healing assessed. The Foxhounds were fed intravenously for seven days post-operatively. Radiographs were made daily. Fecal material was examined when warranted.

Results

The 40% BaSO$_4$ filled BAR implanted in the Beagle began to fragment between the 4th and 7th post-operative day and had been completely excreted by the 12th day. In the Foxhound, the 40% filled BAR had been excreted by the 6th day.

The rings of the 22.5% and 25% BaSO$_4$ filled BAR separated as early as the 6th post-operative day and began fragmenting between the 8th and 11th post-operative days. BAR fragments were found in the fecal material from day 8 through day 16. In no instances were BAR fragments found in the colon in those animals sacrificed on day 17.

Gross examination of the Beagles at autopsy showed excellent serosal healing with a thin band of scar tissue formation. The mucosae of the anastomoses ranged from barely perceptible to moderately indurated and slightly hyperemic. Recovered BAR fragments from all the samples were hard and very brittle. When pressed between the fingers, the fragments crumbled easily.

**Claims**

1. A method for preparing gastro-intestinal anastomosis devices or other surgical tubular support, implant or stenotic devices by molding a mixture of polyglycolic acid with 20 to 40% by weight bariumsulfate based on said mixture and sterilizing the molded product, whereby prior to sterilization said polyglycolic acid or mixture is subjected to a hydrolytic treatment with boiling water at about 100°C during about 30 minutes.

2. A method of Claim 1, characterized in that said polymer or said mixture is subjected to an additional step of re-pelletizing.

3. A method according to Claim 1, characterized in that said barium sulfate is between 20 and 25% by weight.

**Patentansprüche**

1. Verfahren zur Herstellung gastro-intestinaler Anastomose-Vorrichtungen oder anderer chirurgischer tubusförmiger Stützvorrichtungen, Implantate oder Stenosisvorrichtungen durch Formen einer Mischung von Polyglykolsäure mit 20 bis 40 Gew.-% Bariumsulfat, bezogen auf das Gemisch, Sterilisation des geformten Produkts, wobei vor der Sterilisation die Polyglykolsäure oder das Gemisch einer hydrolytischen Behandlung mit siedendem Wasser bei etwa 100°C während etwa 30 Minuten unterworfen wird.

2. Verfahren gemäß Anspruch 1, dadurch gekennzeichnet, daß das Polymere oder das Gemisch einem zusätzlichen Verfahrensschritt der Re-Pelletisierung unterworfen wird.

3. Verfahren gemäß Anspruch 1, dadurch gekennzeichnet, daß das Bariumsulfat zwischen 20 und 25 Gew.-% ausmacht.

**Revendications**

1. Procédé de préparation de dispositifs pour anastomose gastro-intestinale ou d'autres dispositifs tubulaires chirurgicaux de support, à implanter ou de sténose, dans lequel on moule un mélange d'acide polyglycolique avec 20 à 40% en poids de sulfate de baryum par rapport au dit mélange et dans lequel on stérilise le produit moulé, ledit acide polyglycolique ou ledit mélange étant soumis avant la stérilisation à un traitement hydrolytique avec de l'eau bouillante à 100°C environ pendant environ 30 minutes.

2. Procédé suivant la revendication 1, caractérisé en ce que ledit polymère ou ledit mélange est soumis à une étape supplémentaire de regranulation.

3. Procédé suivant la revendication 1, caractérisé en ce que ledit sulfate de baryum est incorporé en une quantité de 20 à 25% en poids.